# FASCICULE DE BREVET EUROPEEN

(11) **EP 3 082 457 B1**
(45) Date de publication et mention de la délivrance du brevet: **08.01.2020**
(21) Numéro de dépôt: 14827823.7
(22) Date de dépôt: 12.12.2014
(51) Int. Cl.: A23L 5/10, G01N 33/12

(54) **PROCEDE DE CUISSON D'UN ALIMENT ET/OU D'UN AUXILIAIRE DE CUISSON ET D'EVALUATION DE LA FORMATION DE COMPOSES NEOFORMES**
VERFAHREN ZUM KOCHEN VON LEBENSMITTELN UND/ODER KOCHZUBEHÖR UND BEURTEILUNG DER BILDUNG VON NEUGEFORMTEN VERBINDUNGEN
METHOD FOR COOKING FOOD AND/OR A COOKING ACCESSORY AND ASSESSING THE FORMATION OF NEOFORMED COMPOUNDS

(30) Priorité: 20.12.2013 FR 1363301
(43) Date de publication de la demande: 26.10.2016
(73) Titulaire: SEB S.A., 69130 Ecully (FR)
(72) Inventeur: CUILLERY, Pascal, F-74210 Faverges (FR); HOFLEITNER, Céline, F-74000 Annecy (FR)
(74) Mandataire: Bourrières, Patrice
(86) Numéro de dépôt international: PCT/FR2014/053302
(87) Numéro de publication internationale: WO 2015/092232

(56) Documents cités:
- WO-A1-2014/009645
- FR-A1- 2 527 916
- US-A1- 2007 288 192
- KARIN THURNER ET AL: "Determination of cholesterol oxidation products in raw and processed beef and pork preparations", EUROPEAN FOOD RESEARCH AND TECHNOLOGY ; ZEITSCHRIFT FÜR LEBENSMITTELUNTERSUCHUNG UND -FORSCHUNG A, vol. 224, no. 6, 30 juin 2006 (2006-06-30) , pages 797-800, XP019488866, SPRINGER, BERLIN, DE ISSN: 1438-2385
- DEL GOBBO LIANA ET AL: "Cooking decreases observed perfluorinated compound concentrations in fish", JOURNAL OF AGRICULTURAL AND FOOD CHEMISTRY., vol. 56, no. 16, août 2008 (2008-08), pages 7551-7559, XP002730054, USAMERICAN CHEMICAL SOCIETY. WASHINGTON. ISSN: 0021-8561
- FIERENS T ET AL: "Effect of cooking at home on the levels of eight phthalates in foods.", FOOD AND CHEMICAL TOXICOLOGY 2012 FLEMISH INSTITUTE FOR TECHNOLOGICAL RESEARCH (VITO), vol. 50, no. 12, 14 septembre 2012 (2012-09-14), pages 4428-4435, XP002730057,
- PITT W C ET AL: "The effect of cooking on diphacinone residues related to human consumption of feral pig tissues.", FOOD AND CHEMICAL TOXICOLOGY., vol. 49, no. 9, 2011, pages 2030-2034, XP002730055, PERGAMON ISSN: 0278-6915
- B. COMMONER ET AL.: "Mutagenic Analysis as a Means of Detecting Carcinogens in Foods", JOURNAL OF FOOD PROTECTION, vol. 41, no. 12, décembre 1978 (1978-12), pages 996-1003, XP008172255, International Association for Food Protection ISSN: 0362-028X

## Description

L'invention concerne la cuisson d'aliments et/ou d'auxiliaire de cuisson et l'évaluation de la formation de composés néoformés à l'issue de cette cuisson/chauffe.

Est concerné le développement d'un procédé de cuisson a priori par contact qui limite efficacement la formation de composés néoformés dans les aliments et/ou les auxiliaires de cuisson tout en assurant une réussite culinaire et en limitant, ou facilitant, l'intervention de l'utilisateur. Une référence par rapport à une cuisson/chauffe standard est notamment prévue.

Il s'agit ici en particulier de proposer une sorte de protocole d'essai qui permette d'apprécier quelle limitation quantifiée des néoformés a pu être atteinte.

Cela concerne notamment la cuisson/chauffe des aliments et/ou auxiliaires de cuisson dans une poêle à induction, et notamment la matière grasse, la viande et le poisson.

Par cuisson d'un auxiliaire de cuisson, on comprend chauffe d'un auxilaire de cuisson lorsque celui-ci est traité seul sans aliment.

Par composés néoformés, on comprend des substances chimiques qui sont produites naturellement dans les aliments pendant la transformation des aliments, la cuisson, le conditionnement ou les autres opérations unitaires. Ils sont absents des aliments bruts et sont formés seulement dans les aliments qui subissent des changements (chimiques) pendant leur transformation, notamment lors de la cuisson.

On comprend dans les néoformés notamment :
- les hydrocarbures aromatiques polycycliques (HAP)
- les produits des réactions de Maillard comme les amines hétérocycliques, l'acrylamide, la carboxymethyllysine (CML) et l'hydroxyméthylfurfural (HMF)
- les produits de dégradation des lipides comme les oxystérols, les acides gras trans et monomères cycliques

Il est ici précisé qu'on doit comprendre par « cuisson standard », une cuisson réalisée avec une température de la surface chauffante (chauffée par un dispositif de chauffage et via laquelle l'aliment cuit) qui augmente globalement dans le temps, tant que la seconde puissance est fournie.

Lors d'une cuisson standard, c'est l'utilisateur qui pilote la cuisson, notamment le réglage de la source de chauffe en observant des indicateurs de transformation des aliments, par exemple l'apparition de fumée de l'huile qui indique que l'huile est suffisamment chaude et qu'il faut baisser la puissance de chauffe.

Concernant la « cuisson régulée », on doit la comprendre comme:
- débutant habituellement à température ambiante (de l'ordre de 20°C), avec éventuellement une préchauffe à vide de la surface chauffante (montée en température de cette surface puis stabilisation autour d'une valeur de consigne),
- se poursuivant avec l'ajout de l'aliment et/ou auxiliaire de cuisson au contact de la surface chauffante (baisse alors en général de la température de ladite surface, puis remontée et stabilisation autour d'une seconde valeur de consigne), éventuellement un retournement de l'aliment (baisse alors en général à nouveau de la température de ladite surface puis remontée et stabilisation autour de la - d'une éventuelle troisième - valeur de consigne). Ainsi, lors d'une cuisson régulée, la température de la surface chauffante est régulée autour d'au moins une température de consigne.

Quant à la « cuisson standard », on doit la comprendre:
- comme précédemment, début typique à température ambiante, éventuellement une préchauffe à vide (montée en température, puissance relativement constante), ajout de l'aliment et/ou auxiliaire de cuisson (légère baisse ou stabilisation de la température puis ré-augmentation de la température, puissance relativement constante), éventuellement un retournement de l'aliment (légère baisse ou stabilisation de la température puis augmentation de la température, puissance de nouveau relativement constante).

Ci-après les cuissons respectivement régulée, suivant a1) et/ou a2), et standard, suivant b1) et/ou b2), en fournissent deux références.

A partir de FR-A-2527916, on connait un procédé de cuisson mettant en œuvre un appareil de cuisson à induction et un article culinaire dont le fond est muni d'un insert ferromagnétique à température de point de Curie déterminé.

Toutefois, ce document ne divulgue pas un procédé mettant en œuvre un article culinaire présentant un fond comportant une zone ferromagnétique adaptée pour faire de préférence découpler la table à induction une fois la température de Curie atteinte, dans le cadre de la gestion de protocole prévue.

KarinThurner et al.:Determination of cholesterol oxidation products in raw and processed pork and beef preparations" Eur. Food Res. Technol. (2007) 224: 797-800, divulgue un procédé de cuisson d'un aliment et de différentes huiles et d'évaluation de la formation de composés d'oxydation du cholestérol.

Ainsi, aucun protocole n'est divulgué visant à apprécier une limitation de formation de néoformés par rapport à une cuisson standard.

L'invention a notamment pour objectif de pallier tout ou partie des inconvénients de l'art antérieur.

En particulier, un objectif est de définir différentes étapes d'un protocole ou méthode permettant de dégager des intérêts toxicologiques d'une cuisson régulée.

Cela permettra notamment de soutenir le bien-fondé d'allégations vis-à-vis de l'apparition de composés néoformés connus pour être toxiques et susceptibles d'être présentées par exemple sur un emballage d'article culinaire ou dans des documents promotionnels.

Un sous-objectif est de dégager les intérêts toxicologiques par rapport à une cuisson standard.

Tout ou partie de ces objectifs est atteint en prévoyant un procédé de cuisson d'un aliment et/ou d'un auxiliaire de cuisson et d'évaluation de la formation de composés néoformés, caractérisé en ce qu'il comprend les étapes suivantes :
- fournir au moins deux échantillons de l'aliment cru et /ou de l'auxiliaire de cuisson non chauffé,
- prédéfinir pour le premier de ces échantillons des temps de cuisson/chauffe pour différentes évolutions de température de la surface chauffante, pour cuire, on pourvoit un article culinaire présentant ladite surface chauffante d'un moyen de détection sensible à la température et interrompant une partie au moins de la transmission d'énergie du dispositif de chauffage vers l'article lorsque la température détectée dépasse une valeur déterminée.
- lors d'une étape de conduite de protocole, on conduit deux protocoles différents de cuisson/ chauffe, respectivement pour l'un et l'autre des échantillons, au contact de la surface chauffante, l'un des protocoles incluant une cuisson régulée en température, l'autre une cuisson non-régulée, chaque protocole étant réalisé pendant ledit temps prédéfini qui lui correspond, et,
- lors d'une étape de dosage des composés néoformés, on réalise:
   * un dosage de composés néoformés contenus dans les deux échantillons cuits/chauffés selon les protocoles de cuisson respectifs et/ou
   * un dosage de composés néoformés contenus dans le premier échantillon cuit/chauffé selon la cuisson régulée et dans l'échantillon initial.
   Pour renforcer la pertinence des résultats des protocoles de cuisson et d'évaluation évoqués, on recommande par ailleurs que :
      - lors de ladite étape de prédéfinition de temps de cuisson, on prédéfinit, pour les échantillons, des temps de cuisson pour différentes évolution d'énergie de chauffe fournies par un dispositif de chauffage et/ou différentes évolutions de température de la surface chauffante,
      - lors de ladite étape de conduite de protocole, on conduit deux protocoles différents de cuisson, respectivement pour l'un et l'autre des échantillons, au contact de la surface chauffante, l'un des protocoles incluant une cuisson régulée en énergie et/ou en température, l'autre une cuisson non-régulée, chaque protocole étant réalisé pendant ledit temps prédéfini qui lui correspond, et,
      - lors de ladite étape de dosage de composés néoformés, on réalise :
         * un dosage de composés néoformés contenus dans les deux échantillons cuits selon les protocoles de cuisson respectifs et/ou
         * un dosage de composés néoformés contenus dans le premier échantillon cuit selon la cuisson régulée et dans l'échantillon initial.

Et même de préférence, pour encore davantage de précision et/ou fiabilité des résultats atteints, on conseille que :
- lors de l'étape de prédéfinition des temps de cuisson, on prédéfinisse un premier et un second temps de cuisson de deux échantillons identiques de l'aliment cru et /ou de l'auxiliaire de cuisson non chauffé, respectivement :
   * pour une première puissance de chauffe régulée ou pour une première évolution de température régulée de chauffe et correspondant respectivement à
      a1) une puissance de chauffe assurée par le dispositif de chauffage, d'abord sensiblement constante puis qui décroit globalement, après une éventuelle augmentation, diminution et stabilisation suite au contact avec l'échantillon,
      a2) une température de la surface chauffante qui d'abord augmente globalement puis se stabilise à une valeur sensiblement constante, après une éventuelle diminution, augmentation et stabilisation suite au contact avec l'échantillon,
   * pour une seconde puissance de chauffe standard ou pour une seconde évolution de température standard de chauffe et correspondant respectivement à :
      b1) une puissance de chauffe assurée par le dispositif de chauffage qui est globalement sensiblement constante dans le temps,
      b2) une température de la surface chauffante qui augmente globalement dans le temps, tant que la seconde puissance est fournie,
- lors de l'étape de conduite des protocoles de cuisson, on cuise les deux échantillons, respectivement :
   * avec la première puissance de chauffe régulée ou la première évolution de température régulée de chauffe, pendant le premier temps de cuisson,
   * et avec la seconde puissance de chauffe standard ou la seconde évolution de température standard de chauffe, pendant le second temps de cuisson,
- lors de l'étape de dosage, on dose des composés néoformés contenus dans chacun desdits échantillons cuits et on dose les mêmes composés néoformés contenus dans l'échantillon.

A priori, et de préférence :
- la comparaison évoquée entre les dosages sera conduite en se rapportant à l'échantillon cru, et/ou
- lors des cuissons, toutes les autres conditions de cuisson non spécifiées ci-avant en référence aux puissances/ températures/temps de cuisson seront identiques entre les cuissons ; ainsi, et par exemple, dans le cas d'aliments solides, on conseille de retourner chaque échantillon à mi-cuisson (mi-durée de cuisson).

Par ailleurs, on appelle ici :
- temps de cuisson, le temps de contact de l'échantillon (aliment et/ou auxiliaire de cuisson) avec la surface chauffée (plaque pour griller, teppanyaki, poêle...), afin de le cuire/chauffer,

- énergie délivrée, l'énergie fournie par le dispositif de chauffe, qu'il s'agisse d'une puissance, d'une intensité, d'une tension électrique (ainsi dans le texte, les termes « énergie » et « puissance » sont synonymes, sauf quand une unité SI ou une courbe d'évolution est mentionnée),
- cuisson à cœur, la cuisson réalisée de l'échantillon (aliment et/ou auxiliaire de cuisson) sensiblement au milieu de celui-ci,
- article culinaire : un ustensile de cuisine dans ou sur lequel on cuit par contact un aliment et/ou auxiliaire de cuisson (plaque pour griller, teppanyaki, poêle...), ou encore un accessoire de cuisine, tel un couvercle ou une spatule.

Un problème peut se poser concernant la manière d'assurer notamment une cuisson régulée performante, en particulier dans le cadre du protocole ici évoqué.

A cette fin, il est proposé:
- qu'on pourvoit un article culinaire ou ladite surface chauffante (laquelle appartient soit à l'article culinaire soit au dispositif de chauffage) d'un capteur de mesure physique parmi une température, un flux de chaleur et un débit de vapeur, puis que, lors de ladite étape de cuisson selon le(s) protocole(s), on enregistre la mesure physique, et qu'on renvoie des données enregistrées vers une électronique de traitement qui régulera automatiquement l'énergie de chauffe et/ou qui informera un utilisateur de la nécessité de réguler la cuisson, et/ou,
- que, pour cuire, on place chaque échantillon dans et au contact d'une poêle avec possibilité d'ajout de matière grasse.

Avantageusement, le dispositif de chauffage étant un appareil de chauffage par induction, on pourvoit l'article d'un matériau magnétique présentant un point de Curie égal à +/- 15°C à ladite température déterminée.

Un problème peut se poser concernant encore le type de cuisson à assurer, pour conduire de façon pertinente et performante le protocole ici évoqué.

A cette fin, on conseille de cuire les échantillons par contact de ces échantillons avec la surface chauffante, à pression ambiante (dans ce cas, donc hors cuisson vapeur, suivant un principe de cocotte-minute, et hors four).

Un problème peut aussi se poser dans la manière de s'assurer si les niveaux de cuisson prévus ont été atteints.

A cette fin, on conseille de se référer à une température à cœur, ou à un autre paramètre physique de l'aliment.

Plus précisément, on propose ainsi en premier lieu :
- de prédéfinir une (la) température à cœur prévue de chaque échantillon pour la cuisson à cœur attendue,
- de relever la température à cœur des échantillons, en fin ou après cuisson, en plaçant une ou plusieurs sondes de températures dans chaque échantillon, et
- de comparer la température relevée à celle prédéfinie.

Dans ce cas, pour une performance des mesures, on recommande :
- d'utiliser comme surface chauffante au moins une plaque pour griller ou une poêle (cuisson par contact donc), de placer à son contact l'échantillon à plat, et de disposer l'une au moins des sondes de températures de manière qu'elle s'étende à mi-hauteur de l'échantillon, parallèlement à une surface de l'échantillon en contact avec la plaque pour griller ou la poêle,
- et/ou, si la taille des échantillons est relativement importante (typiquement faux-filet de bœuf ou darne de saumon de 1,5 cm d'épaisseur), de placer plusieurs sondes de températures dans chaque échantillon, de calculer une moyenne des températures relevées et d'utiliser cette moyenne est pour la comparaison précitée.

En alternative à cette température à cœur, il, est proposé :
- avant cuisson, de prédéfinir un marqueur de cuisson parmi la texture et la couleur de l'échantillon considéré, la modification d'une molécule ou macromolécule prévue dudit échantillon pour une cuisson finale prévue,
- de mesurer le marqueur obtenu en fin ou après cuisson,
- et de comparer les mesures réalisées à celle prédéfinie.

Pour une performance du traitement des résultats/relevés obtenus, on recommande par ailleurs que :
- lors de ladite comparaison entre les valeurs mesurées et celles prédéfinies, on vérifie que l'écart entre elles est inférieur ou égal à 5%, à 40% près, selon le test de Student, et/ou,
- qu'après le dosage des échantillons, une analyse statistique de Student soit conduite, avec un test de Student appairé si les échantillons à comparer sont issus d'un même lot, et/ou,
- qu'avant et après la cuisson, chaque échantillon soit pesé pour calculer une perte en poids de l'échantillon causée par la cuisson (si elle est faible, cette valeur est révélatrice d'une performance de la cuisson en termes limitation de dégradation de l'aliment, de limitation de la formation de composés néoformés et de cuisson peu asséchante).

Un autre problème peut se poser relativement au traitement des échantillons après cuisson, afin de préserver la pertinence des mesures à effectuer.

A cette fin, il est proposé :
- qu'après la cuisson et avant le dosage, les échantillons soient amenés à une température négative, en °C, et/ou
- qu'avant le dosage les échantillons soient broyés s'il s'agit d'aliments solides. Encore un autre problème peut se poser relativement à la manière de conduire les cuissons régulée et/ou standard afin qu'en particulier dans le premier cas, les résultats obtenus soient les plus performants possibles en termes de limitation de formation de composés néoformés et/ou de cuisson peu asséchante.

A cette fin, il est proposé :
- que, pour la cuisson à première puissance de chauffe régulée ou première évolution de température régulée et à 10% près au maximum, on aboutisse à une température de la surface chauffante comprise entre 205 et 215°C, et/ou la puissance de chauffe délivrée est d'abord inférieure à 1800W, pour terminer à moins de 800W, et/ou
- que, pour la cuisson à seconde puissance de chauffe standard ou seconde évolution de température standard de et à 10% près au maximum, on délivre une puissance qui est globalement entre 1100W et 2250W, et/ou on atteigne une température de la surface chauffante d'au moins 250°C à l'issue dudit second temps de cuisson.

D'autres caractéristiques et/ou avantages pourront ressortir de la description complémentaire ci-après, fournie à titre non limitatif, en référence aux dessins annexés, dans lesquels :
- la figure 1 schématise des articles culinaire et accessoire de cuisine, avec une boucle de régulation, avec un chauffage par induction;
- la figure 2 est une vue partielle en coupe verticale d'une poêle adaptée à un chauffage par induction ;
- les figures 3,4 schématisent respectivement un chauffage par une source de gaz et une source électrique ;
- la figure 5 montre de dessous un article culinaire adapté à un chauffage par induction ;
- la figure 6 montre des évolutions d'énergie/température en fonction de durées prédéterminées dans le cadre d'un ou plusieurs protocoles de chauffage/cuisson eux-mêmes prédéfinis ;
- les figures 7,8 montrent une implantation de sondes de température dans un échantillon (la figure 8 est une vue suivant la flèche VIII de la figure 7);
- et la figure 9 schématise le montage de thermocouples utilisables comme moyens de détection.

Le protocole concerné par le mode de réalisation présenté ci-après est basé sur la comparaison de deux modes de cuisson poêlés :
- une cuisson non-régulée, dite standard dans l'exemple (poêle 1 de diamètre 26 cm, obtenue par frappe à chaud, chauffage de la poêle sur un foyer à induction 3). La puissance fournie par la plaque lorsque la poêle est remplie d'eau est comprise entre 1100W et 1855W (3 essais),
- une cuisson régulée dont la température maximale est comprise entre 205 et 215°C (poêle 2 de diamètre 26 cm, obtenue par frappe à chaud avec une coupelle en matériau phytherm 210, chauffage de la poêle sur un foyer à induction 3). La puissance fournie par la plaque lorsque la poêle est remplie d'eau est en moyenne de 1445W (3 essais), soit un 25 débit d'évaporation de 0,49 gramme d'eau par seconde (3 essais). A la température de régulation, la puissance générée par la plaque est inférieure à 800W.

On aurait pu évaluer la formation de composés néoformés en ne comparant qu'une cuisson régulée avec données toxicologiques de l'aliment cru.

Il suffira alors, avant cuisson, de prédéfinir pour au moins un premier des échantillons à tester au moins un temps de cuisson et le protocole de cuisson associé, régulée en énergie et/ou en température au contact de la surface chauffante 10 chauffée par le dispositif de chauffage 3.

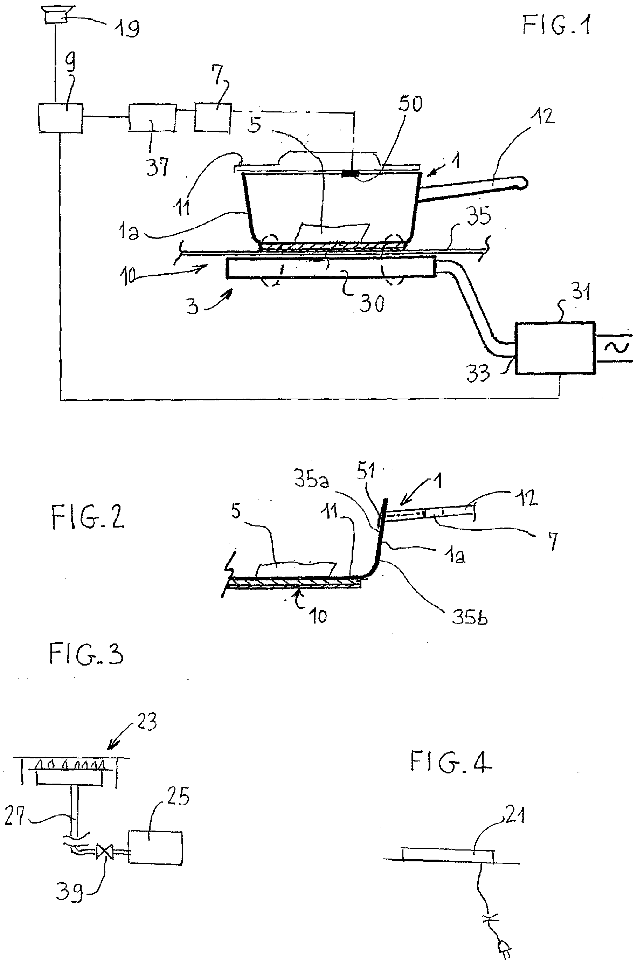

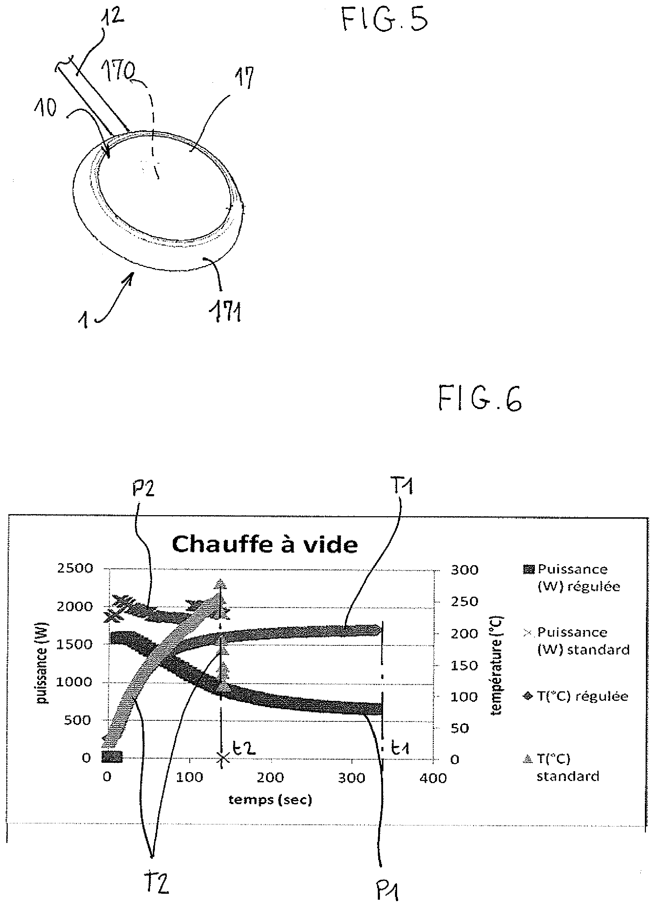

Dans l'exemple, la surface chauffante 10 est le fond de la poêle ou casserole 1 (voir figure 1 ou 5), suivant la cuisson concernée.

En alternative, ce pourrait être par exemple la paroi latérale de la poêle, ou une plaque grill à induction sur laquelle serait directement cuit l'aliment et/ou auxiliaire de cuisson concerné.

Ainsi, on cuira le(s) échantillon(s) 5 par contact de ces échantillons avec la surface chauffante 10, à pression ambiante.

On a ici choisi, pour cuire, de placer chaque échantillon dans et au contact d'une poêle 1, avec possibilité d'ajout de matière grasse. Et cette cuisson qui s'opère par contact avec la surface chauffante est à pression ambiante (atmosphérique).

Avant d'appliquer le protocole retenu, on va donc dans l'exemple fournir ici au moins deux échantillons crus de l'aliment, en l'espèce d'origine animal.

A cet égard, il convient de noter que la formation de composés néoformés dans un aliment au cours de la cuisson peut typiquement dépendre de paramètres comme :
- la composition de la matière première (taux de lipides, protéines, glucides...)
- la teneur initiale en composés néoformés (aliment cru),
- le morceau de l'animal choisi/origine de l'échantillon et de la préparation des échantillons,
- le mode de cuisson,
- le niveau de cuisson (plus ou moins longue, plus ou moins grillé, cuit à cœur...).

Dans l'exemple, on a sélectionné une viande et/ou un poisson et/ou une huile de cuisson.

Pour la viande, on a retenu des tranches (ici de faux-filet) provenant d'un même animal (ou si besoin de nombreuses tranches d'animaux de même race, même âge). On donc obtenu des tranches pratiquement identiques : Les faux-filets ont été raidis (-2°C) puis tranchés de manière à obtenir des tranches de 1,5 à 2 cm d'épaisseur. Les échantillons été numérotés en fonction de leur ordre (de la tête à la queue) et en fonction de s'ils proviennent du morceau droit ou gauche. Pour le poisson, le saumon a été choisi. Les poissons provenait d'un même élevage et étaient quasi du même calibre (taille, poids). Les saumons ont été tranchés à la machine de la tête à la queue pour obtenir des darnes de 1,5 cm d'épaisseur. Les darnes ont été numérotées en commençant par les darnes côté tête. Seules les 12 darnes du milieu ont été gardées (darnes 3 à 14) de manière à minimiser la variation du taux lipidique intra individu. Les darnes ont été coupées en deux pour former des demi darnes. L'arête centrale et la peau ont été enlevées.

De manière à maîtriser la variabilité intra-individu (tête-queue, droite-gauche), les demi-darnes de chaque saumon ont été différenciées en fonction de la droite et la gauche du saumon puis réparties successivement entre la cuisson standard, la cuisson régulée et le cru. Pour les différents saumons, la première demi-darne (darne numéro 3, droite) correspond successivement à une cuisson standard, une cuisson régulée ou un échantillon cru comme illustré dans le tableau suivant :

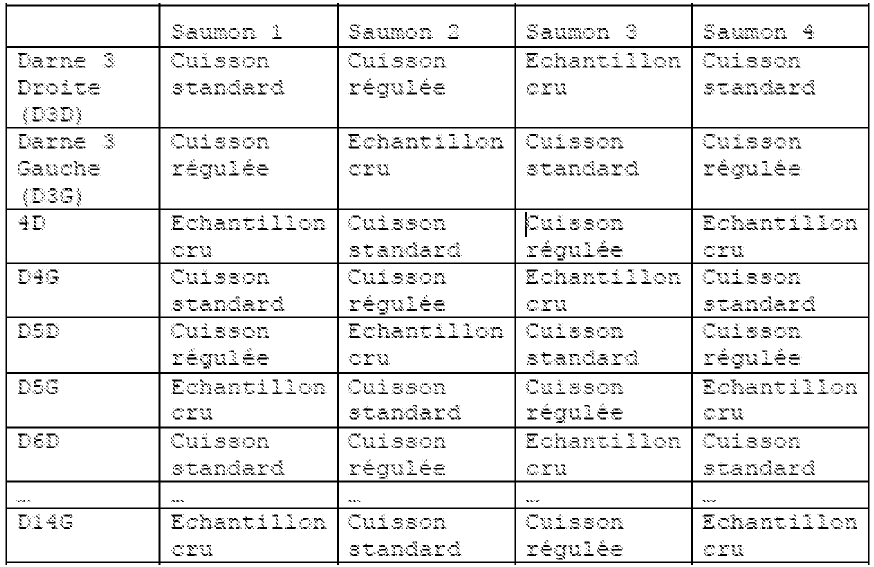

Avant la cuisson les échantillons sont légèrement épongés à l'aide d'un papier absorbant et sont pesés.

Pour l'huile, l'huile de tournesol non oléique a été choisie pour sa tenue en température et sa compatibilité avec une cuisson poêlée ainsi que pour sa faible variabilité de composition entre deux lots. Cependant, un même lot a été préférentiellement utilisé pour les analyses. Les chauffes régulées et standards ont été menées alternativement.

Par ailleurs, avant cuisson selon le protocole concerné, on a donc prédéfini (voir figure 6) un premier t1 et un second t2 temps de cuisson des deux échantillons identiques crus de l'aliment, respectivement :
- pour une première puissance de chauffe régulée P1 figure 2, ou pour une première évolution de température régulée de chauffe T1, et correspondant, comme on le voit, respectivement à :
   * une puissance de chauffe assurée par le dispositif de chauffage 3, d'abord sensiblement constante puis qui décroît globalement, après une éventuelle augmentation, diminution et stabilisation suite au contact avec l'échantillon 5 (évolutions non représentées puisque les courbes sont « à vide »),
   * une température de la surface chauffante 10 qui d'abord augmente globalement puis se stabilise à une valeur sensiblement constante, après une éventuelle diminution, augmentation et stabilisation (à nouveau non représentées) suite au contact avec l'échantillon,
- pour une seconde puissance de chauffe standard P2, ou pour une seconde évolution de température standard T2 de chauffe et correspondant respectivement à :
   * une puissance de chauffe assurée par le dispositif de chauffage 3 qui est globalement sensiblement constante dans le temps,
   * une température de la surface chauffante 10 qui augmente globalement dans le temps, tant que la seconde puissance est fournie.

Toujours avant la cuisson, on a encore pourvu l'article culinaire 1, ici sa surface chauffante 10 (mais avec une cuisson directement sur une plaque à griller cela aurait pu être la plaque elle-même), d'un capteur de mesure physique 5 parmi une température, un flux de chaleur et un débit de vapeur. Grâce à cela on va pouvoir, lors de l'étape de cuisson selon le(s) protocole(s), enregistrer sur l'enregistreur 7 (figure 1) la mesure physique et renvoyer des données enregistrées vers l'électronique de traitement 9 qui régulera automatiquement l'énergie de chauffe et/ou qui informera (via une alerte 19, tel un afficheur ou un message sonore) un utilisateur de la nécessité de réguler la cuisson, selon le protocole en cause.

De fait, on a donc équipé un accessoire d'article culinaire, ici le couvercle 11 de la poêle 1, d'un moyen de détection 50 sensible à un paramètre physico-chimique et interrompant, via par exemple l'électronique de traitement 9 et dans le cadre du protocole, une partie au moins de la transmission d'énergie du dispositif de chauffage 3 vers l'article 1, lorsque le paramètre détecté dépasse une valeur déterminée.

Dans l'exemple de l'apparition de composés néoformés dans l'huile de cuisson, on a choisi de simuler une cuisson poêlée longue, c'est-à-dire une préchauffe jusqu'à 180°C puis 8 minutes de cuisson dans une poêle frappe à chaud de 26 cm de diamètre. Les temps de préchauffe (pour atteindre 180°C mesuré par mesure infra rouge) a été compris entre 1min25 et 1min40. Pour la cuisson régulée, le niveau de puissance de la plaque n'est pas modifié par l'utilisateur. Deux températures de régulation maximale ont été testées : 205 et 215°C. Pour la cuisson standard, le niveau de puissance de la plaque est ajusté de manière à ce que la température monte jusqu'à 240°C et soit maintenue entre 230 et 240°C. 240°C correspondant à la température de fumée de l'huile qui est un indicateur signalant à l'utilisateur que l'huile est suffisamment chaude et qu'il faut baisser la puissance de chauffe.

Il existe de nombreux composés néoformés. Dans le cas de l'huile, il a été décidé d'étudier les polymères de triglycérides qui sont générés par la réactivité des doubles liaisons sous l'effet thermique ainsi que les aldéhydes non volatils issus de la dégradation des hydroperoxydes (composés primaires de l'oxydation de l'huile).

Les polymères de triglycérides sont dosés par chromatographie liquide haute performance décrite dans la norme NF EN ISO 9936 et les aldéhydes non volatils mesurés par l'indice d'anisidine décrit dans la norme NF EN ISO 6885. Pour chaque chauffe, trois répétitions ont été réalisées et un test de Student a été mené pour vérifier la différence statistique des résultats.

| Polymères de triglycérides % | | | | |
|---|---|---|---|---|
| | huile non chauffée | huile après chauffe régulée à 205°C | huile après chauffe régulée à 215°C | huile après une chauffe standard |
| | 0,7 | 3,0 | 3,4 | 5,2 |
| | | 3,0 | 3,7 | 4,8 |
| | | 3,0 | 3,5 | 4,8 |
| Moyenne | | **3,00** | **3,53** | **4,93** |
| Ecart type | | 0,00 | 0,15 | 0,23 |
| Limite la formation par rapport au standard | | **-45,67%** | **-33,07%** | |

| Indice d'anisidine | | | | |
|---|---|---|---|---|
| | huile non chauffée | huile après chauffe régulée à 205°C | huile après chauffe régulée à 215°C | huile après une chauffe standard |
| | 2,3 | 65,3 | 68,6 | 100,8 |
| | | 63,3 | 76,6 | 96,7 |
| | | 64,4 | 72,3 | 95,6 |
| Moyenne | | **64,33** | **72,50** | **97,70** |
| Ecart type | | 1,00 | 4,00 | 2,74 |
| Limite la formation par rapport au standard | | **-34,98%** | **-26,42%** | |

D'autres mesures peuvent être menées sur d'autres aliments comme la viande et le poisson. Dans ce cas, la variabilité des échantillons est maitrisée en calibrant l'épaisseur, la masse des échantillons ainsi que la provenance de la matière première. Le niveau de cuisson est facilement mesurable par mesure de température à cœur, mais peut en complément, ou plutôt en substitution de la mesure de températures 51, on aurait pu, avant cuisson :
- prédéfinir au moins un marqueur de cuisson parmi :
   * la texture (tel : moelleux, croustillant, dureté, croquant, tendreté,...) et la couleur de l'échantillon considéré, ceci étant apprécié au toucher ou visuellement par un opérateur, et
   * via une analyse (physico-)chimique, la modification d'une molécule ou macromolécule (telle : dénaturation, gélatinisation, hydrolyse) prévue de l'échantillon pour la cuisson finale prévue,
- mesurer (ou apprécier) le marqueur obtenu en fin ou après cuisson,
- puis comparer les mesures réalisées à celle prédéfinie.

Plus précisément, on aurait pu ainsi pu conduire :
* une analyse sensorielle avec un panel d'experts (descripteurs prédéfinis),
* une analyse de dénaturation des protéines de l'échantillon (DSC : Differential Scanning Calorimetry
   - détection par calorimétrie différentielle, fluorimétrie,...),
* une analyse de la texture (texturomètre : compression, pénétration, dureté, traction, force à la rupture,...), par exemple un texturomètre TA XT2 ou TA plus une analyse de la couleur (réflectance, systèmes L*a*b,...).

Concernant l'article culinaire 1, s'il s'agit d'une poêle ou plus généralement d'un récipient à poignée(s) de levage, on recommande a priori d'utiliser un article compatible induction à surface chauffante 10, et une source inductive associée comme dispositif de chauffage 3.

Il sera alors aisé, pour l'étape de cuisson régulée, que la surface 10 soit automatiquement régulée, en particulier à une température de 205±10°C.

Pour cela, on recommande, comme illustré figure 5, que l'article culinaire comporte un fond pourvu d'une plaque (un disque dans l'exemple) 17 en acier inoxydable (ferro) magnétique. Ce fond peut présenter une épaisseur d'1 mm environ et être soudé à la face inférieure d'une plaque de fond 170 plus épaisse en aluminium ou cuivre qui peut être elle-même soudée au fond 17l de la carcasse en acier inoxydable formant la calotte creuse de la poêle illustrée.

La surface de la plaque 17 pourra être déterminée de façon à ne plus (ou moins) être détectée par l'inducteur 30 de la table à induction 3 et être ainsi découplée, une fois la température de Curie atteinte (dans le cas précité environ 205°C).

En pratique :
- pour la cuisson régulée, le chauffage débute à température ambiante, puis il y a eu : préchauffe à vide (sans article culinaire et/ou sans aliment), montée en température puis stabilisation autour d'une valeur de consigne), ensuite placement de l'aliment sur la surface de cuisson (avec alors baisse de la température puis remontée et stabilisation autour de la valeur de consigne), et retournement de l'aliment (baisse de la température puis remontée et stabilisation autour de la valeur de consigne).
- pour l'autre cuisson : à nouveau début à température ambiante, préchauffe à vide (montée en température, puissance relativement constante), placement de l'aliment sur la surface de cuisson (avec légère baisse ou stabilisation de la température puis augmentation de la température, avec puissance relativement constante) et retournement de l'aliment (légère baisse ou stabilisation de la température puis augmentation de la température, avec encore puissance relativement constante).

Après cuisson, et avant le dosage, les échantillons peuvent être amenés à une température négative, en °C. On conseille de les congeler pour préservation. Avant dosage, dans le cas d'aliments solides, ils sont en outre de préférence broyés, y compris celui cru.

Concernant le traitement des données, la mesure du poids avant (cru) et après cuisson permet de calculer la perte en poids (en %) engendrée par le mode de cuisson.

Ainsi, de façon générale, conseille t'on qu'après le dosage des échantillons, une analyse statistique de Student soit conduite, avec un test de Student appairé si les échantillons à comparer sont issus d'un même lot.

Les résultats peuvent alors être exprimés comme une limitation de la formation de composés néoformés (exemple : la cuisson régulée limite de X % la formation de composés néoformés).

Les résultats des essais menés ont été les suivants :
Parmi les avantages apportés par la solution proposée ci-avant, on relève que le/chaque protocole d'essai permet:
- de mettre en évidence un bénéfice toxicologique d'un mode de cuisson par rapport à un autre. Il est facilement reproductible et fiable ;
- de mesurer la perte en poids (majoritairement perte en eau). Une différence de perte en poids peut être caractérisée par un bénéfice toxicologique ;
- de maitriser la variabilité de la matière première et apporte une analyse statistique pour vérifier la différence (statistiquement différent à 5%, à 40% près) des modes de cuisson.

Le protocole peut aussi être adapté pour mesurer l'impact du mode de cuisson sur la dégradation des huiles de cuisson.

De ce qui précède, on peut conclure que, pour évaluer la limitation de la formation de composés néoformés d'au moins un aliment, on utilisera au moins deux échantillons crus de l'aliment et, pour le premier (au moins), on prédéfinira au moins un temps de cuisson et un protocole de cuisson régulée. Le second échantillon où le dosage des composés néoformés sera effectué pourra demeurer cru.

Deux protocoles différents de cuisson, respectivement pour l'un et l'autre des (au moins deux) échantillons, au contact de la surface chauffante permet de comparer cuisson régulée en énergie et/ou en température et cuisson non-régulée. Et leurs particularités sont respectées, puisque chaque protocole est réalisé pendant le temps prédéfini qui lui correspond.

Concernant la surface chauffante 10 (appelée aussi surface de cuisson), elle appartiendra soit au dispositif de chauffage 3 soit à l'article culinaire. Le dispositif 3 pourra assurer un chauffage par induction. Ainsi, il pourra comprendre la bobine ou inducteur 30 (figure 1) alimentée par l'onduleur 31 tirant son énergie du courant électrique fourni par le secteur. L'onduleur 31 fournit sur sa sortie 33 un courant électrique alternatif ayant de préférence une forte intensité et sous faible tension. Au-dessus de cette bobine est disposée une plaque isolante 35, par exemple en verre, sur laquelle peut être posée, au droit de la bobine 1, une casserole ou poêle à fond magnétique 10.

Par exemple si la plaque verre 35 est remplacée par un grill, telle une plaque ondulée électriquement isolante, la surface chauffante 10 appartiendra au dispositif de chauffage 3.

La surface chauffante 10 pourrait aussi être une plaque chauffée par-dessous par :
- une résistance électrique 21 raccordée à une source d'alimentation, telle le secteur, (voir figure 4), ou
- un brûleur à gaz 23 alimenté en gaz comburant (typiquement air) et en gaz combustible par une source 25 par exemple propane, via la conduite 27 (voir figure 3).

On peut aussi imaginer que la surface chauffante 10 soit la paroi latérale 1a d'un article culinaire, porteuse d'une résistance électrique.

Concernant maintenant le capteur 50 ou 51 de mesure physique, il pourra donc être sensible à une température ou un flux de chaleur ou un débit de vapeur.

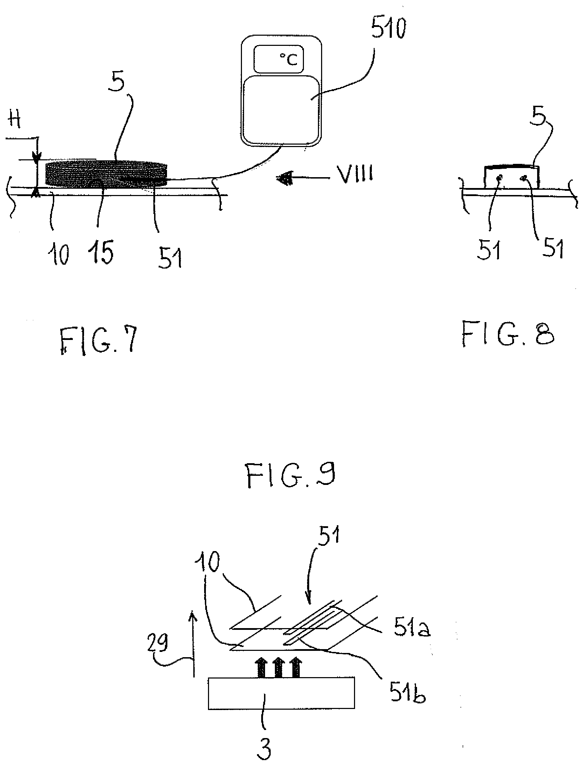

Un thermocouple pourra être un capteur de température performant. Il peut être placé sur ou dans l'article culinaire 1 (au niveau de la couche d'aluminium ou de l'inox pour un tel article du type à calotte et poignée(s) : poêle, casserole...), sur la surface de cuisson 10, au niveau de la poignée 12 de l'article ou encore au niveau d'un couvercle (tel 11), comme schématisé figures 1,2 ou 5, ou autre ustensile ou accessoire qui entre en jeu 10 au cours de la cuisson (spatule...). Comme montré figure 9, deux thermocouples 51a, 51b placés successivement, typiquement en superposition, dans le sens du flux de chaleur 29 permettent de détecter une différence de températures à un temps donné et donc de définir le capteur de flux de chaleur 51.

Dans le cas d'un capteur de débit de vapeur, on peut prévoir, comme montré figure 2, que l'article culinaire, tel le haut de la jupe de l'article 1, ou un autre ustensile ou accessoire de cuisine (couvercle, spatule) inclut une structure (telle la jupe 1a de l'article ou le bloc couvercle 11) présentant une première et une deuxième faces 35a, 35b opposées l'une à l'autre, et au moins ce moyen de détection 51, ici sensible aux composés volatils issu de la cuisson. Le moyen de détection 51 est relié au dispositif de traitement 7 apte à émettre un signal s'il y a une détection desdits composés volatils. Par souci de facilité de mise en œuvre et d'emploi, le moyen de détection peut être intégré au moins à la première face 35a, laquelle sera agencée pour être au contact ou en regard d'un aliment. On recommande en outre que cette première face soit nue ou revêtue d'une couche de revêtement antiadhésif en matériau sol-gel ou en résine fluorocarbonée formant un réseau continu fritté. Quant au moyen de détection 51, on conseille qu'il comprenne :
- un matériau actif apte à modifier ses propriétés de conduction électrique en présence des composés volatils);
- au moins deux éléments de connexion assurant la connexion entre le matériau actif et le dispositif de traitement.

Concernant son fonctionnement, on prévoit favorablement que ce moyen de détection et le dispositif de traitement 7 forment un circuit électrique où le matériau actif présentera une résistivité variant en présence des composés volatils et, si un courant électrique traversant le dispositif de traitement devient différent d'un seuil prédéterminé, le dispositif de traitement émettra un signal. Une boucle de régulation permettant la cuisson régulée et couplée au dispositif de traitement pour notamment adapter l'énergie électrique délivrée, en fonction des données/signaux émis par le détecteur 51 de composés volatils, est envisagée.

Lors de l'étape de cuisson selon le(s) protocole(s), la mesure physique/chimique/physico-chimique concernée sera enregistrée en mémoire 37, et les données enregistrées pourront être envoyées vers l'électronique de traitement 9 qui pourra:
- réguler automatiquement l'énergie de chauffe (par exemple en modifiant l'énergie délivrée par la résistance électrique 21 ou par l'onduleur 30, ou encore en agissant sur une vanne 39 interposée sur la conduite 27),
- et/ou qui informera l'utilisateur de la nécessité de réguler la cuisson, par exemple par le moyen d'alerte ou d'affichage 19 ; voir figure 1.

## Revendications

1. Procédé de cuisson d'un aliment et/ou d'un auxiliaire de cuisson et d'évaluation de la formation de composés néoformés, **caractérisé en ce qu'**il comprend les étapes suivantes :
- fournir au moins deux échantillons (5) de l'aliment cru et /ou de l'auxiliaire de cuisson non chauffé,
- prédéfinir pour le premier de ces échantillons des temps de cuisson/chauffe pour différentes évolutions de température de la surface chauffante (10), pour cuire, on pourvoit un article culinaire (1) présentant ladite surface chauffante (10) d'un moyen de détection (50,51) sensible à la température et interrompant une partie au moins de la transmission d'énergie du dispositif de chauffage (3) vers l'article (1) lorsque la température détectée dépasse une valeur déterminée.
- lors d'une étape de conduite de protocole, on conduit deux protocoles différents de cuisson/chauffe, respectivement pour l'un et l'autre des échantillons (5), au contact de la surface chauffante (10), l'un des protocoles incluant une cuisson régulée en température, l'autre une cuisson non-régulée, chaque protocole étant réalisé pendant ledit temps prédéfini qui lui correspond, et,
- lors d'une étape de dosage des composés néoformés, on réalise :
* un dosage de composés néoformés contenus dans les deux échantillons (5) cuits/chauffés selon les protocoles de cuisson respectifs et/ou
* un dosage de composés néoformés contenus dans le premier échantillon cuit/chauffé selon la cuisson régulée et dans l'échantillon initial.

2. Procédé selon la revendication 1, **caractérisé en ce que** le dispositif de chauffage (3) étant un appareil de chauffage par induction (3), on pourvoit l'article d'un matériau magnétique (17) présentant un point de Curie égal à +/-15°C à ladite température déterminée.

3. Procédé selon l'une des revendications 1 à 2, **caractérisé en ce qu'**on cuit les échantillons (5) par contact de ces échantillons avec la surface chauffante (10), à pression ambiante.

4. Procédé selon l'une des revendications 1 à 3, **caractérisé en ce que** :
- avant cuisson, on prédéfinit un marqueur de cuisson parmi la texture et la couleur de l'échantillon (5) considéré, la modification d'une molécule ou macromolécule prévue dudit échantillon pour une cuisson finale prévue,
- on mesure le marqueur obtenu en fin ou après cuisson,
- on compare les mesures réalisées à celle prédéfinie.

5. Procédé selon l'une des revendications 1 à 3, **caractérisé en ce que** :
- avant cuisson, on prédéfinit une température à cœur prévue de chaque échantillon (5) pour la cuisson à cœur attendue, ou on prédéfinit une valeur cible d'un paramètre, notamment la couleur ou la texture,
- on mesure la température à cœur des échantillons (5), en fin ou après cuisson/chauffe, en plaçant une ou plusieurs sondes de températures (51) dans chaque échantillon (5) ou on mesure la valeur du paramètre en fin de cuisson/chauffe, et
- on compare la température mesurée ou la valeur mesurée du paramètre choisi, à la valeur cible prédéfinie.

6. Procédé selon la revendication 5, **caractérisé en ce qu'**on utilise comme surface chauffante (10) une poêle (1), on place à son contact l'échantillon (5) à plat, et on dispose l'une au moins des sondes (51) de manière qu'elle s'étende à mi-hauteur de l'échantillon, parallèlement à une surface de l'échantillon (5) en contact avec la plaque (17, 170) pour griller ou la poêle (1).

7. Procédé selon la revendication 5 ou 6, **caractérisé en ce qu'**on réalise pour un même échantillon plusieurs mesures, on calcule une moyenne et cette moyenne est utilisée pour ladite comparaison.

8. Procédé selon l'une des revendications 5 à 7, **caractérisé en ce que** lors de ladite comparaison entre les valeurs mesurées et celles prédéfinies, on vérifie que l'écart entre elles est inférieur ou égal à 5%, à 40% près, selon le test de Student.

9. Procédé selon l'une des revendications 1 à 8, **caractérisé en ce qu'**avant et après la cuisson, chaque échantillon (5) est pesé pour calculer une perte en poids de l'échantillon causée par la cuisson.

10. Procédé selon l'une des revendications 1 à 9, **caractérisé en ce qu'**après la cuisson et avant le dosage, les échantillons (5) sont amenés à une température négative, en °C.

11. Procédé selon l'une des revendications 1 à 10, **caractérisé en ce qu'**avant le dosage les échantillons (5) sont broyés.

12. Procédé selon l'une des revendications 1 à 11, **caractérisé en ce qu'**après le dosage des échantillons (5), une analyse statistique de Student est conduite, avec un test de Student appairé si les échantillons à comparer sont issus d'un même lot.

13. Procédé selon l'une des revendications 1 à 12, **caractérisé en ce que**, pour la cuisson régulée et à 10% près au maximum, on aboutit à une température de la surface chauffante (10) entre 205 et 215°C, et/ou la puissance de chauffe délivrée est d'abord inférieure à 1800W, pour terminer à moins de 800W.

## Patentansprüche

1. Verfahren zum Kochen eines Lebensmittels und / oder eines Kochhilfsmittels und zur Bewertung der Bildung von neu geformten Bestandteilen, **dadurch gekennzeichnet, dass** es die folgenden Schritte umfasst:
- Bereitstellen von mindestens zwei Proben (5) des rohen Lebensmittels und / oder nicht erhitzten Kochhilfsmittels,
- Vordefinieren von Koch- / Heizzeiten für die erste dieser Proben bei unterschiedlichen Entwicklungen der Temperatur der Heizfläche (10), wobei zum Kochen ein Küchenartikel (1) vorgesehen wird, der die Heizfläche (10) eines temperaturempfindlichen Erfassungsmittels (50, 51) vorweist und mindestens einen Teil der Energieübertragung von der Heizvorrichtung (3) auf den Artikel (1) unterbricht, wenn die erfasste Temperatur einen bestimmten Wert überschreitet.
- während eines Schritts der Protokollausführung werden zwei verschiedene Koch- / Heizprotokolle für die eine bzw. die andere der Proben (5) in Kontakt mit der Heizfläche (10) ausgeführt, wobei eines der Protokolle ein in der Temperatur geregeltes Kochen, das andere ein nicht in der Temperatur geregeltes Kochen umfasst, wobei jedes Protokoll während der vorgegebenen Zeit, die ihm entspricht ausgeführt wird, und
- während eines Schrittes der Dosierung der neu geformten Bestandteile ausgeführt wird:
* eine Dosierung der neu geformten Bestandteile, die in beiden Proben (5) enthalten sind, die gemäß den jeweiligen Kochprotokollen gekocht / erhitzt wurden, und / oder
* eine Dosierung der neu geformten Bestandteile, die in der ersten Probe enthalten sind, die gemäß dem geregelten Kochen und in der Anfangsprobe gekocht / erhitzt wurde.

2. Verfahren nach Anspruch 1, **dadurch gekennzeichnet, dass**, wenn die Heizvorrichtung (3) ein Induktionsheizgerät (3) ist, der Artikel mit einem magnetischen Material (17) versehen wird, das einen Curie-Punkt gleich +/- 15 ° C bei der ermittelten Temperatur vorweist.

3. Verfahren nach einem der Ansprüche 1 bis 2, **dadurch gekennzeichnet, dass** die Proben (5) durch Kontakt dieser Proben mit der Heizfläche (10) bei Umgebungsdruck gekocht werden.

4. Verfahren nach einem der Ansprüche 1 bis 3, **dadurch gekennzeichnet, dass**:
- vor dem Kochen ein Kochmarker aus der Textur und der Farbe der betrachteten Probe (5) vordefiniert wird, wobei die Modifikation eines Moleküls oder Makromoleküls der Probe für ein endgültiges vorgegebenes Kochen vorgegeben ist,
- der erhaltene Marker wird am Ende oder nach dem Kochen gemessen,
- die durchgeführten Messungen werden mit der vordefinierten verglichen.

5. Verfahren nach einem der Ansprüche 1 bis 3, **dadurch gekennzeichnet, dass**:
- vor dem Kochen eine jeder Probe (5) vorgegebene Kerntemperatur für das erwartete Kernkochen vordefiniert wird, oder ein Sollwert eines Parameters, insbesondere die Farbe oder die Textur, vordefiniert wird,
- die Kerntemperatur der Proben (5) wird am Ende oder nach dem Kochen / Erhitzen gemessen, indem in jede Probe (5) eine oder mehrere Temperatursonden (51) eingesetzt werden, oder der Parameterwert am Ende des Kochens / Heizens wird gemessen, und
- die gemessene Temperatur oder der gemessene Wert des ausgewählten Parameters wird mit dem vordefinierten Wert verglichen.

6. Verfahren nach Anspruch 5, **dadurch gekennzeichnet, dass** als Heizfläche (10) eine Pfanne (1) verwendet wird, und die Probe (5) flach in Kontakt gebracht wird, und mindestens eine der Sonden (51) so angeordnet ist, dass sie sich auf halber Höhe der Probe parallel zu einer Oberfläche der Probe (5) in Kontakt mit der Platte (17, 170) zum Grillen oder der Pfanne (1) erstreckt.

7. Verfahren nach Anspruch 5 oder 6, **dadurch gekennzeichnet, dass** für dieselbe Probe mehrere Messungen durchgeführt werden, ein Durchschnitt berechnet wird und dieser Durchschnitt für den Vergleich verwendet wird.

8. Verfahren nach einem der Ansprüche 5 bis 7, **dadurch gekennzeichnet, dass** während des Vergleichs zwischen den gemessenen Werten und den vordefinierten überprüft wird, dass die Differenz zwischen ihnen weniger als oder gleich 5%, ungefähr 40 %, gemäß dem Student's Test beträgt.

9. Verfahren nach einem der Ansprüche 1 bis 8, **dadurch gekennzeichnet, dass** jede Probe (5) vor und nach dem Kochen gewogen wird, um einen Gewichtsverlust der Probe zu berechnen, der durch das Kochen verursacht ist.

10. Verfahren nach einem der Ansprüche 1 bis 9, **dadurch gekennzeichnet, dass** die Proben (5) nach dem Kochen und vor der Dosierung auf eine negative Temperatur in °C gebracht werden.

11. Verfahren nach einem der Ansprüche 1 bis 10, **dadurch gekennzeichnet, dass** die Proben (5) vor der Dosierung gemahlen werden.

12. Verfahren nach einem der Ansprüche 1 bis 11, **dadurch gekennzeichnet, dass** nach der Dosierung der Proben (5) eine statistische Student's Analyse mit einem gepaarten Student's Test durchgeführt wird, wenn die zu vergleichenden Proben von einer einzelnen Charge stammen.

13. Verfahren nach einem der Ansprüche 1 bis 12, **dadurch gekennzeichnet, dass** für das kontrollierte Kochen und bei 10% nahe dem Maximum eine Temperatur der Heizfläche (10) zwischen 205 und 215 °C erreicht wird, und / oder die abgegebene Heizleistung zunächst weniger als 1800 W beträgt und bei weniger als 800 W endet.

## Claims

1. Method for cooking food and/or a cooking oil and for evaluation of the formation of neoformed compounds, **characterised in that** it comprises the following steps:
- provide at least two samples (5) of the raw food and/or the unheated cooking oil;
- predefine cooking/heating times for the first of these samples, for different changes in the temperature of the heating surface (10), for cooking, a cooking utensil (1) having said heating surface (10) of a detection means (50, 51) sensitive to the temperature and interrupting a part at least of the transmission of energy from the heating device (3) to the utensil (1) when the detected temperature exceeds a determined value,
- during a protocol execution step, two different cooking/heating protocols are performed, for one and for the other samples (5) respectively, in contact with the heating surface (10), one of the protocols including cooking with regulated temperature, the other with unregulated cooking, each protocol being performed during said predefined time corresponding to it, and,
- during a proportioning step of the neoformed compounds:
* the neoformed compounds contained in the two cooked/heated samples (5) are proportioned according to the corresponding cooking protocols, and/or
* the neoformed compounds contained in the first cooked/heated sample are proportioned according to the regulated cooking and in the initial sample.

2. Method according to claim 1, **characterised in that** the heating device (3) being an induction heating appliance (3), the utensil is made of a magnetic material (17) having a Curie point equal to said determined temperature within +/- 15°C.

3. Method according to claim 1 or 2, **characterised in that** the samples (5) are cooked by contact of these samples with the heating surface (10), at ambient pressure.

4. Method according to one of claims 1 to 3, **characterised in that**:
- before cooking, a cooking marker is predefined from among the texture and the colour of the sample (5) considered, modification of a defined molecule or a macromolecule of said sample for the required final cooking,
- the marker obtained at the end of cooking or after cooking is measured,
- the measurements made are compared with the predefined value.

5. Method according to one of claims 1 to 3, **characterised in that**:
- before cooking, an internal temperature is predefined for each sample (5) for the required degree of internal cooking, or a target value of a parameter, particularly the colour or the texture, is predefined,
- the internal temperature of the samples (5) is measured at the end of or after cooking/heating, by placing one or several temperature probes (51) in each sample (5) or the value of the parameter is measured at the end of cooking/heating, and
- the measured temperature or the measured value of the chosen parameter is compared with the predefined target value.

6. Method according to claim 5, **characterised in that** a pan (1) is used as the heating surface (10), the sample (5) is placed flat in contact with the pan, and at least one of the probes (51) is arranged such that it extends at mid-height of the sample, parallel to a surface of the sample (5) in contact with the griddle (17, 170) or the pan (1).

7. Method according to claim 5 or 6, **characterised in that** several measurements are made for the same sample, an average value is calculated, and this average is used for said comparison.

8. Method according to one of claims 5 to 7, **characterised in that** during said comparison between the measured values and the predefined values, it is checked that the difference is less than or equal to 5%, within 40%, according to the Student test.

9. Method according to one of claims 1 to 8, **characterised in that** before and after cooking, each sample (5) is weighed for use in calculating a loss of weight of the sample caused by cooking.

10. Method according to one of claims 1 to 9, **characterised in that** after cooking and before proportioning, the samples (5) are brought to a negative temperature in °C.

11. Method according to one of claims 1 to 10, **characterised in that** the samples (5) are ground before proportioning.

12. Method according to one of claims 1 to 11, **characterised in that** after proportioning of the samples (5), a Student statistical analysis is performed, with a matched Student test if the samples to be compared originate from the same batch.

13. Method according to one of claims 1 to 12, **characterised in that**, for regulated cooking and within 10% of the maximum, the temperature of the heating surface (10) obtained is between 205 and 215°C and/or the output heating power is less than 1800W in the beginning, and terminates at less than 800W.
